(19) **Europäisches Patentamt**
**European Patent Office**
**Office européen des brevets**

(11) **EP 3 660 506 A2**

(12) **EUROPEAN PATENT APPLICATION**

(43) Date of publication:
**03.06.2020 Bulletin 2020/23**

(21) Application number: **19216720.3**

(22) Date of filing: **17.10.2014**

(51) Int Cl.:
*G01N 33/20* (2019.01)   *B82Y 30/00* (2011.01)
*C07K 17/00* (2006.01)   *C12N 15/10* (2006.01)
*C12Q 1/68* (2018.01)   *C25D 11/02* (2006.01)
*C40B 50/14* (2006.01)   *G01N 1/02* (2006.01)
*G01N 1/10* (2006.01)   *C07K 17/14* (2006.01)
*C25D 5/44* (2006.01)   *C25D 7/00* (2006.01)
*C25D 11/22* (2006.01)   *C25D 11/24* (2006.01)
*C25D 11/26* (2006.01)   *C25D 11/30* (2006.01)
*C25D 11/34* (2006.01)   *G01N 33/543* (2006.01)
*C12Q 1/6816* (2018.01)   *C25D 11/08* (2006.01)
*C25D 11/16* (2006.01)   *C25D 11/10* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.10.2013   US 201361892786 P**

(62) Document number(s) of the earlier application(s) in
accordance with Art. 76 EPC:
**14854796.1 / 3 058 363**

(71) Applicant: **9163-0384 Québec Inc.
Saguenay, Québec G7H 5H6 (CA)**

(72) Inventors:
• **ARSENAULT, Steve**
**Laterrière, Québec G7N 1N1 (CA)**
• **GAUDET, Daniel**
**Chicoutimi, Québec G7H 3S1 (CA)**
• **RIVARD, Daniel**
**St-Honoré de Chicoutimi, Québec G0V 1L0 (CA)**
• **DUMONT, Maxime**
**Chicoutimi-Nord, Québec G7G 4B7 (CA)**
• **LAMBERT, Jocelyn**
**Roberval, Québec G8H 3J4 (CA)**

(74) Representative: **Icosa**
**83 avenue Denfert-Rochereau
75014 Paris (FR)**

Remarks:
This application was filed on 16-12-2019 as a
divisional application to the application mentioned
under INID code 62.

(54) **TRACEABLE METALLIC PRODUCTS AND METALLIC SUPPORT FOR NANOSTORAGE**

(57)   The invention relates to traceable metallic products, methods of uses and methods of making same. The metallic products may be made traceable for integrity purposes, identification purposes, counterfeit avoidance and the like. The invention also relates to metallic supports for nanostorage of various compounds and samples.

**EP 3 660 506 A2**

**Description**

**FIELD OF INVENTION**

The invention relates to traceable metallic products. The metallic products may be made traceable for integrity purposes, identification purposes, counterfeit avoidance and the like. The invention also relates to metallic supports for nanostorage of various compounds and samples.

**BACKGROUND OF INVENTION**

In many industries, traceability of materials and avoidance of counterfeit products is very important, particularly where safety is a critical issue. For instance, better systems are being developed to trace meat products from the farmer, to the slaughterhouse, to the grocery store and finally to the consumers. Drug manufacturers often put a label comprising a hologram to their products so that a consumer can easily differentiate original brand products and potential counterfeit products. In the aviation industry tracking is also critical because the use of counterfeit aircraft and avionic parts has serious implications for flight safety, national resilience and security.

There is thus a need for traceability solutions that can be effectively applied without imposing prohibitively high costs. So far, no one has ever proposed traceable metallic products comprising a porous surface layer formed by anodization where the porous surface layer comprises at least one traceable biological compound. U.S. Patent No. 5,124,172 discloses the use of specific anodizable color-generating metals with a porous anodic film of aluminum coated with antibodies or proteins, but those metallic support are devised for different purposes, particularly the detection or testing of samples taken from patients.

Another interesting avenue for porous anodized metallic products is nanostorage. The number of samples devoted for scientific research is continuously growing while the volume of materials and samples is increasingly smaller. As the number and types of samples continue to grow, there is a need to develop solutions to maximize storage of all these samples in the smallest possible volume and at the lowest cost, while maintaining the integrity and availability of the samples for later analysis or use. However, metallic support for nanostorage is still uncommon. U.S. Patent No. 7,736,724 proposes to manufacture nanobaskets having a diameter of about 1 nanometer to about ten nanometer by sputter-coating techniques of various substrates such as $Al_2O_3$. U.S. Patent No. 7,361,471 proposes using beads of aluminum oxide ($Al_2O_3$) to isolate and store nucleic acids, the nucleic acids tightly binding to the electropositive charges of the beads. However, these patents do not teach impregnating an anodized metallic support with a product to be stored.

There is thus a need for traceable metallic products and for methods of obtaining and tracking the same. There is also a need for methods and products for nanostorage on a metallic support.

The present invention addresses these needs and other needs as it will be apparent from review of the disclosure, drawings and description of the features of the invention hereinafter.

**BRIEF SUMMARY OF THE INVENTION**

The invention relates to traceable metallic products which may be made traceable for integrity purposes, identification purposes, counterfeit avoidance purposes and the like. The invention also relates to a metallic support for nanostorage (or nanobanking) of various compounds and samples such as biological compounds, biochemical compounds, chemical compounds and the like.

One particular aspect of the present invention concerns a traceable metallic product comprising a porous surface layer formed by anodization, the porous surface layer comprising at least one traceable biological compound.

Another aspect of the present invention concerns a method for obtaining a traceable metallic product, comprising the steps of:

- providing an anodized metallic product having a porous surface layer formed by anodization; and
- impregnating a region of said porous surface layer with at least one traceable biological compound.

Accordingly, a related aspect of the invention concerns the use of at least one traceable biological compound for tracking an anodized metallic product comprising a porous layer, wherein the at least one traceable biological compound is found inside pores of the surface layer.

In embodiments, the at least one traceable biological compound is selected from the group consisting of nucleic acids, peptidic molecules (peptides, proteins, lipoprotein, glycosylated-protein), lipids, mono and polysaccharides, hormones, vitamins, and derivatives thereof.

Another aspect of the present invention concerns a traceable piece of aluminum comprising a porous surface layer formed by anodization, the porous surface layer comprising nucleic acid molecules inside pores of the surface layer.

Another aspect of the present invention concerns a method for tracking a porous metallic product having a porous surface layer formed by anodization, comprising:

assigning at least one predetermined traceable compound to a porous metallic product to be tracked;
impregnating a region of the porous surface layer with the at least one predetermined traceable compound;
tracking the porous metallic product later in time by detecting and/or identifying the at least one predetermined traceable compound and by correlating the presence and/or identity of the at least one predetermined traceable organic compound with the assigning; a positive detection or identification providing a positive verification and traceability of the metallic product to be tracked. In particular embodiments, said detecting and/or identifying comprises recovering said predetermined traceable compound.

The at least one predetermined traceable compound may be detectable, identifiable and/or utilizable. The at least one predetermined traceable compound may be recoverable from said porous surface layer for later detection and/or identification. In embodiments, the traceable compound is detected following an exposure to heat or cold, to an exposure to light, following its isolation and/or following a chemical reaction in situ. In particular embodiments, the predetermined traceable compound is a biological compound selected from the group consisting of nucleic acids, peptidic molecules, lipids, mono and polysaccharides, hormones, and vitamins. In other embodiments, the predetermined traceable compound is a chemical compound selected from the group consisting of reactants, colorants, fluorescent products, phosphorescent products, antibacterials, antivirals, antibiotics, antifungals, odorants, and gustative compounds.

According to the various aspects of the invention, the metallic product may be selected from the group consisting of aluminum, titanium, zinc, magnesium, niobium, tantalum and anodizable alloys thereof.

In various embodiments, the porous surface layer further comprises an electrodeposit of at least one metal selected from the group consisting of silver, gold, copper, nickel, zinc, tin, cadmium, palladium and platinum.

In some embodiments, the porous surface layer is sealed. Sealing or clogging may be carried out before, simultaneously or after impregnation.

In some embodiments, the porous surface layer is dyed or colored. The dying or coloration may be made before, simultaneously or after the impregnation.

In some embodiments, the metallic product according to the invention consists of, or is a component of, an article of manufacture. Accordingly, an additional aspect of the invention concerns articles of manufacture incorporating a traceable metallic product and/or a traceable piece of aluminum as defined herein. In embodiments, the article of manufacture is selected from the group consisting of plane parts, automotive vehicle parts, train parts, boat parts, electronic and computer components, military-related products, medical devices, jewelry, art works, products labels (e.g. food, drugs), keys, food containers, credit cards, money, collectibles, casino equipment, and any additional articles where traceability may be useful for sorting, tracking, identification, verification, authentication, anti-theft, anti-counterfeit, security/antiterrorism, forensics, or other similar purposes.

In selected embodiments, the traceable metallic product and/or the article of manufacture consists of a metallic support for nanostorage of compounds and/or samples. Accordingly, an additional aspect of the invention concerns a method for nanostorage on a metallic support, comprising the steps of:

- providing an anodized metallic support having a porous surface layer formed by anodization; and
- impregnating a region of the porous layer with a product to be stored.

In particular embodiments, the product to be stored is a biomedical sample, a biochemical sample, a chemical sample, or an environmental sample. In particular embodiments, the biological material to be stored is selected from the group consisting of whole blood, blood constituents, urine, cell extracts, nucleic acid molecules, proteins, lipids, saccharides, metabolomics, and vitamins. In particular embodiments, the biological sample comprises biological materials selected from the group consisting of nucleic acids, proteins, metabolites, lipids, blood, serum, plasma, urine, cell extracts, DNA, RNA, proteins, lipids, saccharides, vitamins, metabolomics. In particular embodiments, the chemical sample to be stored comprises organic molecules, inorganic molecules, drugs, reactants. The porous surface layer may further comprise a dye and/or a sample stabilizer which is selected according to a desired biological material or sample to be stored.

An advantage of the present invention is that it provides simple, cheap, fast and efficient means for tracking all kinds or products and more particularly metallic products, and for the nanostorage of various kinds of samples and compounds.

Additional aspects, advantages and features of the present invention will become more apparent upon reading of the following non-restrictive description of preferred embodiments which are exemplary and should not be interpreted as limiting the scope of the invention.

## BRIEF DESCRIPTION OF THE DRAWINGS

**Figure 1** is a schematization of a process for making an anodized metallic product with nanostorage properties according to one embodiment of the invention.

**Figure 2** is a schema illustrating detection of PCR amplification products following their migration on a separation gel, according to Example 2.

**Figure 3** is a picture of an anodized aluminum 2x2 cm plate of about 0.2 mm thick, the picture revealing detection by the Maillard reaction of soy proteins and soy lecithins impregnated into the plate according to Example 3.

**Figure 4** is a picture of an agar plate microbial lawn showing inhibition zones resulting from diffusion of the antimicrobial benzalkonium chloride from an anodized metallic disk, according to Example 6.

**Figure 5** is a picture of anodized aluminum disks colored with bromophenol blue according to Example 8. Deposition of a drop of an acetic acid solution on the disk resulted in a change of color from blue to yellow at the location where the solution was deposited (B, right picture).

## DETAILED DESCRIPTION OF THE INVENTION

### A) Traceable metallic products and methods of using same

One particular aspect of the present invention address the need for traceability of materials and avoidance of counterfeit products.

Accordingly, one aspect of the invention concerns a traceable metallic product having a porous surface layer formed by anodization, the porous surface layer comprising at least one traceable biological compound.

One related aspect of the invention concerns a method for obtaining a traceable metallic product, comprising the steps of:

- providing an anodized metallic product having a porous surface layer formed by anodization; and
- impregnating a region of said porous surface layer with at least one traceable biological compound.

Another related aspect of the invention concerns a method for tracking a metallic product having a porous surface layer formed by anodization, comprising:

assigning at least one predetermined traceable compound to a porous metallic product to be tracked;
impregnating a region of the porous surface layer with said at least one predetermined and traceable compound;
tracking said porous metallic product later in time by detecting and/or identifying said at least one predetermined traceable compound and by correlating the presence and/or identity of said at least one predetermined traceable organic compound with said assigning; wherein a positive detection or identification provides a positive verification and traceability of the metallic product to be tracked.

According to preferred embodiments of that method, the porous surface layer comprises nanopores and the traceable compound may be any compound which may be introduced and carried in the nanopores of the porous surface layer.

In some embodiments, the traceable compound comprises a chemical compound selected from the group consisting of reactants (ions, pH-indicator, oxydo-redox indicators), colorants (e.g. dyes, pigments, inks, paint, colored chemicals), fluorescent products (e.g. ethidium bromide, SYBR green, vitamin B2, anthracene, stilbene, etc.), phosphorescent products (e.g. A1204, Calcium sulfide, alkaline earth metal silicate, etc.), chemiluminescent products (e.g. luminol), products impacting positively or negatively growth and or survival of microorganisms (e.g. antibacterials such as quaternary ammonium, antivirals, antibiotics, and antifungals, nutrients, etc.), odorants (e.g. fragrance, menthol, β-mercaptoethanol, hydrogen sulfide, ammonia or any product detectable by dogs), gustative compounds (salts, sweeteners, bitterness (coffee, cocoa, chicory), sourness (acids)), or products whose presence may trigger an exothermic or endothermic reaction and such that the resulting cold or heat can be felt by touch (e.g. cold-pack like compounds (endothermic reactants) or hot-pack like compounds (exothermic reactants)).

In some embodiments, the traceable compound comprises at least one traceable biological compound. The traceable biological compound may be any natural molecule which can be isolated from a living organism or any synthetic molecule deriving therefrom. Examples of traceable biological compounds include, but are not limited to, nucleic acids, peptidic molecules (peptides, proteins, lipoproteins, glycosylated-protein, etc.), lipids, mono and polysaccharides, hormones, vitamins, derivatives thereof and any compound composed of these. Accordingly, a related aspect of the invention concerns the use of at least one traceable biological compound for tracking an anodized metallic product comprising a porous layer.

The traceable compound may be detectable, identifiable and/or utilizable. In a preferred embodiment the traceable compound is recoverable from the porous surface layer for detection, identification and/or utilization purposes. Indeed, traceable metallic products according to the invention may be useful for sorting, tracking, identification, verification, authentication, anti-theft, anti-counterfeit, security/antiterrorism, forensics, or for other similar purposes.

The selection of the traceable compound will vary according to numerous factors including, but not limited to, the type of metal to track, the environmental conditions to which the traceable metallic product will be exposed, the desired level of stealth or the level of visibility for the traceable compound, the intended users or intended consumers, the size and intended uses for the traceable metallic product, the size and intended uses of the article(s) of manufacture incorporating the traceable metallic product, the desired longevity for the traceability, the compatibility of the traceable biological compounds (if more than one are present or required), the need for recovery for later detection, identification and/or utilization purposes, and the need for a plurality of distinct "codes" or "labels" for the traceable metallic product(s) to be made.

For instance, nucleic acids offer the possibility of an unlimited number of distinct codes since each nucleic acid molecule comprises a particular sequence which can serve as a unique identifier. An infinite number of nucleic acid molecules of different sizes and sequences can thus be isolated and/or synthesized. As used herein, the term **"nucleic acid"** or **"nucleic acid molecule"** refers to a molecule comprising two or more nucleotides (adenine, cytosine, guanine, thymine, uracil or any other natural or synthetic nucleotide), either single or double stranded, in a either a linear or circular form. Examples of nucleic acid molecules include, but are not limited to, single- or double-stranded DNA, genomic DNA, single-stranded oligonucleotides, single- or double-stranded RNA, amplified PCR products, plasmids, probes, primers, xenonucleics acids (e.g. HNA, TNA, GNA, CeNA, LNA, PNA), etc. The nucleic acid may consist of molecules comprising an identical sequence or it may consist of a plurality of nucleic acid molecules comprising different sequences. According to one embodiment of the invention, an individual could chose to have particular objects or personal items tagged with its own DNA or with nucleic acid molecules comprising its personal genetic code such that these objects be unique and traceable (visibly or not) to him or her.

Thus, it is possible according to the principles of the invention to uniquely label or track any desired item, such as large or small objects, and the present invention can theoretically be used for an endless number of applications including security and law enforcement applications, military applications, manufacturing applications, consumer goods, etc. For instance, one particular use is for the identification of different lots of metal or parts.

The traceable metallic product may be any metallic product comprising a porous surface layer formed by anodization. For instance, the metallic product may be selected from aluminum, titanium, zinc, magnesium, niobium, tantalum and anodizable alloys thereof. The traceable metallic product can be of any size, either very small (e.g. microbeads, a thin foil), or very large (a piece of equipment of a large machine or transportation vehicle, the beam of a building). Examples of articles of manufacture incorporating a traceable metallic product according to the invention include, but are not limited to, flexible foil tags or labels (e.g. anodized aluminum foil tags), aircraft and avionic parts, automotive vehicle parts, train parts, boat parts, electronic and computer components, firearms, military-related devices or equipment, medical devices, jewelry, art works, explosives, buildings, documents, secure notes, products labels and packaging (e.g. food, drugs, blister packs), keys, food containers, credit cards, money, collectibles, casino equipment (machines, cards, dice, etc.) etc.

The traceable compound(s) may be detected using any suitable method or technique. For instance, the traceable compound may be detected following an exposure to heat or cold, by an exposure to light, following isolation (e.g. amplification, electrophoresis, chromatography, migration, chemical or biochemical reaction, purification), or following a chemical reaction in situ (e.g. contacting with antibodies or a chemical reactant) or by any other suitable method.

The traceable compound according to the invention can be made easily visible or almost undetectable. For instance, the traceable compound may be a colorant (e.g. a dye, a pigment, etc.) such that one can easily see its presence and/or identity. It may also be invisible (e.g. because of its particular nature) or hidden somewhere on a small or large region of any given item (e.g. the whole surface of the item may contain the traceable compound or it may be present in only a pinpoint predefined specific location on the item). Products with a hidden traceable compound(s) according to the invention may be very difficult, if not impossible, to counterfeit.

The traceable compound(s) can be made visible or detectable (permanently or not) under given circumstances such as light exposure, exposition to water, exposition to a given temperature level, exposition to a certain gas, following exposition to a particular chemical (e.g. pH indicator), by using an enzymatic reaction, etc. For instance, when exposed to a temperature of about 110-120°C, sugars react with amino acids thereby resulting in the appearance of a brown coloration (i.e. Maillard reaction). Nucleic acid molecules may become visible to the naked eye when in contact with particular chemicals (e.g. ethidium bromide, SYBR Green) and exposed to UV light or blue light. Nucleic acid molecules may also be detected using various techniques of molecular biology such as sequencing, amplification and the like.

Various thermochromic substances exist, these substances will change color due to a change in temperature (typically between -10°C and 65°C). Those skilled in the art can refer to the common general knowledge such as Wikipedia™ (see for instance http://en.wikipedia.org/wiki/Thermochromism incorporated herein by reference) for identifying suitable substances like leuco dyes or other substances such as inorganic compounds that undergo phase transitions or exhibits charge-transfer bands near the visible region. Another example are polydiacetylene compounds which may polymerized together following a change (e.g. elevation) in temperature, thereby provoking an irreversible change in color.

Therefore, the principles of the invention may find unlimited applications to various industries. For instance, it may be possible to easily assess whether a metallic part of a plane has reached a critical undesired temperature, a physical stress, or an exposure to certain chemicals, thereby resulting in the coloration of said part. For many industries (food, drug, storage) it may be possible to easily assess whether a given product has reached a certain elevated temperature (e.g. above -10°C, or above 0°C or above 4°C) during transport or storage. The principles of the invention may be used to obtain and/or verify the identity of various items for miscellaneous purposes such as anti-theft and/or anti-counterfeit (e.g. label on a drug package, casino equipment, bank notes and money), security/antiterrorism (guns, military equipment), forensics, etc.

As will be explained in more detail hereinafter, according to particular embodiments, the traceable metallic product may consist of a metallic support for nanostorage.

## B) Metallic product for nanostorage

One particular aspect of the present invention addresses the need to provide a support enabling the nanostorage of a large number of samples and compounds from different sources. The metallic support according to the invention may be advantageous for the storage of large number of different types of samples, without modifying or changing the support. The metallic support of the invention may be advantageous for maximizing storage of a huge number of samples in the smallest possible volume at low cost, while maintaining the integrity and availability of the samples for later analysis or use. Theoretically, it may be possible, according to the invention, to store more than 100 million samples per square mm, i.e. one individual sample in each one of the nanopores of the anodized surface (with pores of about 100 nm diameter). The metallic support of the invention may be used for short time storage (seconds, minutes or hours) or for storing and keeping the material for long periods of time (e.g. days, weeks, months, years).

One particular aspect concerns a method for nanostorage on a metallic support, comprising the steps of:

- providing an anodized metallic support having a porous surface layer formed by anodization; and
- impregnating a region of the porous layer with a product to be stored.

In one preferred embodiment, the metallic support consists of an anodized metallic product having a porous surface layer formed by anodization. The metallic product may be selected from a suitable metal which can be anodized according to the invention, including, but not limited to aluminum, titanium, zinc, magnesium, niobium, tantalum and anodizable alloys thereof (e.g. aluminum alloys and others).

The samples, materials or compounds to be stored according to the invention can be of different types including, but not limited to, biomedical samples, biochemical samples, chemical samples, and environmental samples. Examples of biomedical samples or biochemical samples include, but are not limited to, nucleic acids, proteins, metabolites, lipids, blood, serum, plasma, urine, cell extracts, DNA, RNA, proteins, lipids, saccharides, vitamins, metabolomics, etc.). Examples of chemical samples include, but are not limited to, organic and inorganic molecules, drugs, reactants, etc. The samples, materials or compounds to be stored can be of different sources including, but not limited to, living organism (e.g. humans, animals, plants, microorganism, etc.), or from the environment (e.g. water samples, soil samples, aqueous air samples, etc.). The samples may be stored separately or mixed in the same nanopores, as needed. Accordingly, the invention further relates to a metallic support for nanostorage of biological samples and/or chemical compounds, and the like. A particular example is an anodized aluminum plate of 4 cm x 8 cm for storing 384 different DNA samples.

Very large numbers of samples may be stored on a very small surface according to the invention. In some embodiments, one may store 1, 10, 50, 100, 500, 1000, 2000, 2500, 5000, 10 000, 100 000 or more samples per $cm^2$. As mentioned herein before, it is theoretically possible to store more than 100 million samples per square mm according to the present invention. For instance, the present invention is amenable to microarray techniques for depositing or "printing" tiny droplets of a given product (e.g. nucleic acids) on the porous metallic surface. Examples include, but are not limited to, commercially available robots such as nano-plotter 2.0E™ or Genetix Q™ array 2 which may print up to 2500 droplets per $cm^2$. The droplets can be as small as 0.5 nL to 10 nL. Similarly, if a small surface of the metallic support according to the invention (e.g. 1 cm$^2$) is impregnated with a single drop of a given product, the invention provides for recovery of a large number of tiny samples from the same original drop for later analysis (e.g. about 2500 samples using current microarray equipment). As the technology evolves, it will be possible to print and recover a greater number of samples per cm$^2$.

Optionally, the metallic support may be washed or rinsed to remove any sample not impregnated in the nanopores. In one embodiment, the porous surface layer is rinsed with 70% ethanol, wiped with a cloth and rinsed quickly with water or rinsed several times with water, to be sure that samples are stored in the nanopores and not on the surface of the anodized layer.

The samples may be recovered from the metallic support using any suitable method. In one embodiment, a droplet of a suitable solvent is deposited on the porous surface of the metallic support and the droplet is allowed to stand on the surface for a sufficient amount of time to allow the sample to exit the nanopores by capillary action, diffusion etc. and be captured by the solvent. Those skilled in the art will be able to select appropriate solvents and recovery methods. For instance, the solvent may be selected from tap or distilled water, an alcohol, a buffer solution, a chemical, a solution permitting to disintegrate any seal or clog of the nanopores, by diffusion on an agar plate, etc. It may also be conceivable according to the present invention to analyze or study the stored sample in situ in the metallic support, i.e. without recovery.

The porous surface layer may further comprise a stabilizer (e.g. DMSO, EDTA) which is selected according to a desired biological material or sample to be stored. The sample stabilizer may provide for a greater conservation of the compounds, molecules and/or samples to be stored.

The porous surface layer may also be sealed or clogged as described hereinafter. The porous surface layer may further comprise a sealant (e.g. gel, chemical compound) to completely plug the outside surface of the pores, to protect or hermetically seal the stored material, and/or to avoid diffusion of the stored material.

The choice of the metal or alloys for making the metallic support, the decision to seal or not, the presence or absence of a plug or sealant, the presence or absence of coloration, the presence or absence of a stabilizer, etc. may depend on various factors, including but not limited to, the desired shape and size of the final product, the type and nature of the sample to be stored, the intended duration of the storage, the number of samples to be stored on a given area, the techniques being used for the impregnation and/or recovery, the working environment, etc.

The metallic support for nanostorage according to the invention may be formatted to different shapes and products, including but not limited to, tubes, plates, supports, baskets, films, sheets, folders, files, etc. In one embodiment, the metallic support consists of aluminum plates having a thickness of about 0.1 mm to about 10 mm.

## C) Method of manufacture

Related aspects of the invention concern methods for obtaining a traceable metallic product and methods for obtaining a metallic support suitable for nanostorage.

Anodization

As indicated herein, the metal support may be selected from aluminum, titanium, zinc, magnesium, niobium, tantalum and anodizable alloys thereof. Anodization of such metals is well known to those skilled in the art. According to the invention, anodization results in the formation of a porous surface layer which is typically, harder, stronger, more brittle, and which is more adherent, than for non-anodized metallic products. In some embodiments, the porous surface layer formed by anodization has a thickness ranging from about 1 $\mu$m to about 150 $\mu$m, or ranging from between 2 $\mu$m to about 35 $\mu$m, or ranging from between 10 $\mu$m to about 20 $\mu$m. The pores are preferably nanopores and they may have a diameter ranging from 5 nm to about 100 nm. Those skilled in the art will understand that the size (i.e. thickness and diameter) of the nanopores may vary depending on various factors, including the quantity of samples, the type of samples and quality of samples to store in it.

It is within the skill of those in the art to anodize various anodizable metals and alloys. Examples of possible techniques include, but are not limited to, type I (chromic acid), type II (sulfuric acid), type III (sulfuric acid hardcoat), and other types of anodization such as chromic-sulfuric acid, oxalic acid, organic acid, phosphoric acid, malonic acid, alkalin, and any anodization method or technique resulting in the formation of a porous surface layer. According to particular embodiments, the aluminum substrate is anodized in a 15 % v/v stirred sulfuric acid solution. A current of 1.5 Amps (13 volts) is applied to the aluminum piece for 44 minutes (given the selected size of the anodized piece, this allows an anodic/cathodic area ratio of 3/1 (the anode is anodized aluminum with a surface area 3 times the cathode surface) (the ratio can be from 1/1 to 4/1)). This leads to a layer of anodization of approximately 20 $\mu$m. The temperature of the solution is maintained between 21°C and 23 °C.

Prior to anodization, the metallic product may be subjected to one or more pre-treatment steps such as degreasing, electropolishing, etching, etc. according to procedures known in the art. In one particular embodiment involving aluminum, aluminum is degreased with acetone; etched with 10% weight/vol NaOH for 2 min at 50-60°C; neutralized in

35% vol/vol $HNO_3$ for 30 sec. at room temperature; and submitted to a P2 etch treatment for 10 min at 50-60°C with 33% v/v sulfuric acid and ferrite (Russell and Garnis, 1977, Chromate-Free Method of Preparing Aluminum Surfaces for Adhesive Bonding. An Etchant Composition of Low Toxicity, Army Armament Research and Development Center, Dover NJ, Large Caliber Weapon Systems Lab).

Impregnation

Incorporation of the traceable compound or incorporation of the product to be introduced or to be stored into nanopores of the anodized metal may be carried out using any suitable method known to those skilled in the art. According to the invention, a region of the porous surface layer of the metallic product is impregnated with the traceable compound or product to be stored. As used herein, "impregnating" or "impregnated" refers to any mechanism by which a compound, sample or product enters into nanopores of the porous surface layer of the anodized metal. For instance, the compound may be deposited directly on a region of the porous surface layer so it can penetrate the pores of the anodized metal by adsorption, by capillary action, by suction, by diffusion, by evaporation, by pressure, etc. For instance, a suction phenomenon could be created by heating the air inside nanopores, by depositing the sample on the surface layer and by letting the nanopores cool such that the volume inside the nanopores diminishes and the sample is "sucked" inside the nanopores. In some embodiments, the sample is simply deposited on the surface of the porous layer and allowed to dry or to evaporate. In another embodiment, the metallic support is immerged or soaked, totally or partially, in the sample. In another embodiment, a product to be stored is pressed against the anodized metal. The metallic support may be impregnated with the sample for a very short time (a few seconds or less) or for longer periods (e.g. minutes, hours, days, or more). The metallic support may be impregnated once or multiple times with the same or with different samples.

Dying and coloration

According to some embodiments, the porous surface layer may be dyed or colored. The invention encompasses any dying or coloration procedure compatible with a traceable metallic product and/or metallic support for nanostorage according to the present invention. Preferably, the dying or coloration is subsequent to anodization. Electrodeposition may be used to color and/or improve the visual aesthetic properties of the anodized metallic support or product and, according to some embodiments, the porous surface layer further comprises an electrodeposit of at least one metal. Various metals can be electrodeposited according to the invention, including, but not limited to, silver, gold, copper, nickel, zinc, tin, palladium, cadmium, platinum and combinations thereof. Electrodeposition (also known as electroplating) is well known to those skilled in the art and it may be used for instance on aluminum to provide lightfast colors (e.g. bronze shades and pale champagne to black).

Alternatively, the color may be produced integral to the coating during the anodization process by using organic acids mixed with a sulfuric electrolyte and a pulsed current. It is also possible to dye the unsealed porous layer of the metal in lighter colors and then splashing darker color dyes onto the surface. Another approach comprises impregnation of the anodized metal in a dying solution. In other embodiments, the dying step is carried out before, simultaneously or after impregnating the porous layer with the at least one traceable compound. For nanostorage purposes, the dying step may be carried out before, simultaneously or after impregnation of the product to be stored.

In some embodiments, the dying or coloration is for aesthetic purposes. In some embodiments, the dying or coloration is for differentiating miscellaneous metallic products or support and may serve as a coding system, for instance to serve as an indicator of the depth and width of the nanopores, to serve as an indicator of the types being stored, to serve as an indicator of the position of the metallic product in a larger device (e.g. particular part in a turbine), etc.

In some embodiments, electrodeposition can be used mainly to color, to improve the visual aesthetic properties, to differentiate and/or to identify the anodized metal support. Various metals can be electrodeposited according to the invention for coloration purposes including, but not limited to, silver, gold, copper, nickel, zinc, tin, palladium, cadmium, platinum and combinations thereof.

Sealing or clogging

In some embodiments, the porous surface layer is sealed or clogged. The sealing may be carried out before, simultaneously or after the impregnation or incorporation of the compound or product into the pores. The sealing may serve different purposes. For instance, it may be used to ensure the traceable compound or the product to be stored stay inside the nanopores.

Various treatment combinations are conceivable for achieving sealing. For instance, one can simultaneously impregnate and seal by soaking the anodized metallic product or support in a solution comprising the traceable compound or product to be stored. In one embodiment, sealing is carried out simultaneously with product impregnation by heating the anodized metallic product or support at about 50°C to 100°C. Another option is to impregnate the anodized metal with the compound or product, then seal the anodized metal with steam at room temperature or in an autoclave. In one embodiment, sealing is achieved after impregnation by treating the anodized metal under pressurized vapor (for instance in an autoclave, with steam at 97°C to 140°C) or with chemical treatment (e.g. salts) at 30°C to 50°C. In another embodiment, the impregnated anodized metal is submitted to pressurized vapor at about 97°C to 130°C.

Those skilled in the art will readily appreciate that the sealing or clogging will have an impact on the incorporation and diffusion of the traceable compound or product to be stored. The more the metallic support is sealed, the slower the speed of diffusion in and out the nanopores, thereby impacting the incorporation and recovery. In one embodiment, the sealing is carried out before impregnation and to control (i.e. reduce) the size of the pores and thereby controlling the total quantity and/or the size of the product or traceable compound to be impregnated (e.g. to allow penetration of only small or short nucleic acids). In one embodiment, sealing is carried out after impregnation and it serves to modulate (i.e. decrease) the speed of diffusion of the product or traceable compound out of the nanopores. In various embodiments, the metallic support is sealed at about 1%, 5%, 10%, 25%, 50%, 75% or at 100%. Those skilled in the art will know how to determine suitable sealing values according to various factors (e.g. types of metal, types of traceable compounds or products to be stored, intended use, durability etc.) and they will know how to assess the level of sealing achieved using various methods.

In embodiments in which the anodized metal is not sealed, the recovery of stored material can be made more easily, e.g. with water or with a buffer by simple diffusion. In embodiments in which the anodized metal is sealed, recovery can be more difficult and can be made with suitable chemicals, such as NaOH. For instance, NaOH can break the anodized layer of aluminum ($Al_2O_3$) to release the incorporated sample. In some embodiments, compounds or samples such as nucleic acids are recovered with distilled water or a TE buffer when the anodized metal is not sealed, whereas they are recovered with NaOH when the support is sealed.

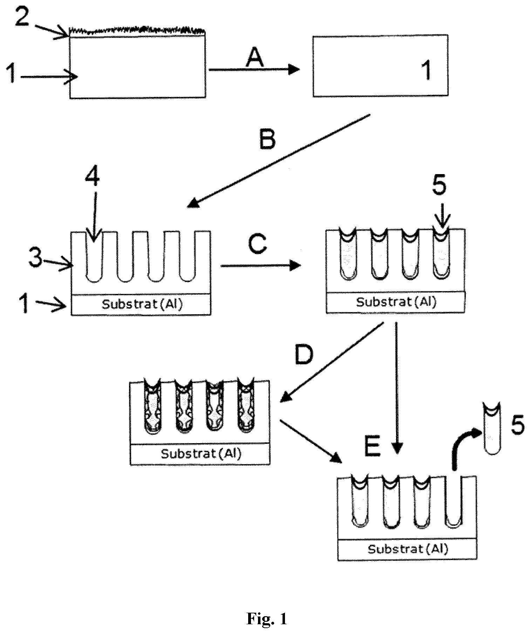

**Figure 1** summarizes the making of a suitable metallic support for nanostorage and the storage of samples therein according to one particular embodiment of the invention. Briefly at Step A: an anodizable metal substrate (1) (e.g. a sheet of aluminum) is degreased with acetone to remove impurities (2) from the surface of the substrate (1); Step B: the metal substrate (1) is anodized resulting in the formation of a porous surface layer (3) comprising nanopores (4); Step C: nanopores are used for the nanostorage of different types of samples (5). Step D: optionally, the pores (4) of the porous surface layer (3) are sealed, either partially or completely to prevent exit of samples. Step E: the samples (5) are recovered for detection, identification and/or utilization. When necessary, the seal is removed for recovering the sample.

Those skilled in the art will recognize, or be able to ascertain using no more than routine experimentation, numerous equivalents to the specific procedures, embodiments, claims, and examples described herein. Such equivalents are considered to be within the scope of this invention and covered by the claims appended hereto. The invention is further illustrated by the following examples, which should not be construed as further limiting.

## EXAMPLES

### EXAMPLE 1: Preparation of anodized aluminum

Anodization

Aluminum was used as an exemplary metallic product and exemplary metallic support according to the invention. Anodization was used to increase the thickness of the natural oxide layer on the surface of aluminum plates and for creating a surface layer comprising nanopores. Aluminum plates of the 1000 to 6000 series were used throughout this study. Plates of 0.07 mm to 3 mm thickness were cut into pieces of various desired shapes. The resulting aluminum pieces were degreased with acetone; etched with 10% weight/vol NaOH for 30 sec to 2 min at 50-60°C; neutralized in 35% vol/vol $HNO_3$ for 30 sec at room temperature; submitted to a P2 etching (33% v/v sulfuric acid and ferrite) for 10 min at 50-60°C and electrochemically oxidized at room temperature in 15% vol/vol sulfuric acid solution at 1.5 amps by $dm^2$ for 11 to 44 minutes (the time depending of the desired thickness of the anodization layer) prior to rinsing with distilled water.

A standardized duration for the anodization was used to obtain a desired thickness. It is known that the thickness is dependent on the duration of the anodization. A theoretical thickness was calculated according to the following equation* :

$$\textit{Oxide thickness (µm) = 0.3 x current density (A/dm}^2\textit{) x anodizing time (min)}$$

*Equation taken from: Anodizing and coloring of aluminum alloys (2002), S. Kawai editor, Publisher: ASM International,

p.170

<u>Sealing of the nanopores</u>

Depending on the testing experiments, the pieces of anodized aluminum were submitted or not to sealing or clogging. Theoretically, the required sealing time corresponds to 2 min for each $\mu$m of oxide thickness (see chapter 9, p67 of Satoshi Kaway, Anodizing and coloring of aluminum alloys, (2002), Finishing Publications, 159 pages). In the present embodiments, excess of the theoretical 100% sealing was obtained by submitting the anodized surface to a thermal treatment under humidity conditions for a period of time equivalent to the duration of the period of anodization (i.e. 44 min of sealing corresponding to an excess of the 40 min required in the literature for an anodization layer of 20 $\mu$m). Therefore, for aluminum pieces that were anodized for 44 min, it was predicted that 44 min will be necessary for sealing 100% of the pores. Because the reaction is mostly linear, it was extrapolated that a sealing treatment of 11 min will result in 25% sealing of the pores.

As described herein before, sealing or clogging may be carried out using any suitable method known in the art. In the examples described herein the anodized aluminum pieces were sealed by exposure to steam (Example 9) and by exposure to steam under controlled pressure (in an autoclave; Example 2).

**EXAMPLE 2: Storage, detection and recovery of nucleic acids**

Ten $\mu$l of double-stranded human genomic DNA (extracted from blood, about 35 Kbp) and small single-stranded oligonucleotides (20-23 base pairs), each at a concentration of 200 ng/$\mu$l in TE 1X buffer were deposited at the surface of different types of aluminum disks, namely anodized, anodized and sealed (with steam) or non-anodized (negative control). The samples were allowed to dry for 15 min then washed with 70% ethanol (12 times for 30 sec) and rinsed with water (5 sec) in order to completely remove any non-incorporated nucleic acids. A solution of ethidium bromide (1% w/v, 100 $\mu$l) was then applied on the surface of the aluminium disks for 5 min, and the disks were washed with ethanol 70% (12 times x 30 sec) and rinsed under water for 5 sec. In one of the tests, the disks were sealed between the deposition of the nucleic acids and the coloration with ethidium bromide. In another test, the disks were sealed after the deposition of the nucleic acids and the ethidium bromide coloration.

Under ambient light, all aluminum disks looked the same, with no visible trace of nucleic acids or coloration. However, as reported in **Table 1,** under UV lighting it was possible to detect both genomic DNA and smaller oligonucleotides that were incorporated into the anodized surface of the disks. As expected, the nucleic acids could not be detected when the anodized surface was sealed prior to the deposition of the nucleic acids, suggesting that both the nucleic acids and the ethidium bromide did not penetrate into the anodized surface of the disks. Intensity was weaker when sealing was performed between the deposition of the nucleic acids (i.e. application on a unsealed surface) and the coloration with ethidium bromide, suggesting that sealing reduced the penetration of the ethidium bromide into the pores. As expected, nucleic acids could not be detected on the non-anodized disks as those disks do not comprises a porous surface layer.

<u>Table 1</u>: **Successful incorporation and detection of nucleic acids in anodized aluminum***

| | Non-Anodized | Non-Anodized + Nucleic acid + ethidium bromide | Anodized | Anodized + nucleic acid + ethidium bromide | Anodized + sealing + nucleic acid | Anodized + Nucleic acid + ethidium bromide | Anodized + Nucleic acid + sealing |
|---|---|---|---|---|---|---|---|
| Genomic DNA | - | - | - | + + + | - | + + + | + |
| Oligonucleotides | - | - | - | + + + | - | + + + | + |

*\* Detection under UV light. (-) : no detection; (+): weak detection; (+++): strongest detection.*

Next, an experiment was carried out to demonstrate that it was possible to recover the nucleic acid molecules from the nanopores and that these nucleic acids were functional. Three different eluents were tested for the recovery: NaOH, water or an aqueous buffer.

Briefly, a drop of about 7,5 $\mu$L of distilled water or of an aqueous buffer (TE 1x) was deposited on a surface of about 4 mm$^2$ of anodized aluminum disks comprising impregnated nucleic acids (either genomic DNA described above, or 7,5 $\mu$l of 100 $\mu$M oligos referred to hereinafter as the GK11_F primer (21 nucleotides) or the GK8 R primer (23 nucleotides). The disks were placed in a closed humid environment (about 90% to 100% humidity) and the nucleic acids were allowed to diffuse out of the porous surface into the water or buffer. After 120 min the deposited drops were recovered with a pipette for analysis of their nucleic acids content.

NaOH was also tested as a potentially more efficacious eluent. Indeed, NaOH was expected to "break" the surface of the anodized aluminum and permit a greater diffusion than water or a simple buffer. A drop of 2.5 M NaOH was deposited on a surface of about 1 mm$^2$ of anodized aluminum comprising the impregnated nucleic acids. After 1 min the drop was recovered with a pipette and the NaOH was removed by diffusion on ice for 90 min in 2% agarose, 50 mM sucrose column. After 90 min the sample was recovered from the column.

All the recovered samples were subjected to PCR amplification and amplified fragments were detected by high resolution capillary electrophoresis in a gel using a QIAxcel™ apparatus. The samples supposed to contain genomic DNA were amplified following addition of GK11_F and GK11_R primers. The samples supposed to contain the primers (GK11_F or GK8_R) were amplified following addition of the second missing primer and the genomic DNA as the template. The four GK primers are for different exon (i.e. exon 8 and exon 11) of the glycerol kinase gene. Amplification of GK11 with the GK11_F and GK11_R primers lead to the amplification of a 219 base pairs fragment and amplification of GK8 with the GK8 F and GK8 R primers lead to the amplification of a 286 base pairs fragment.

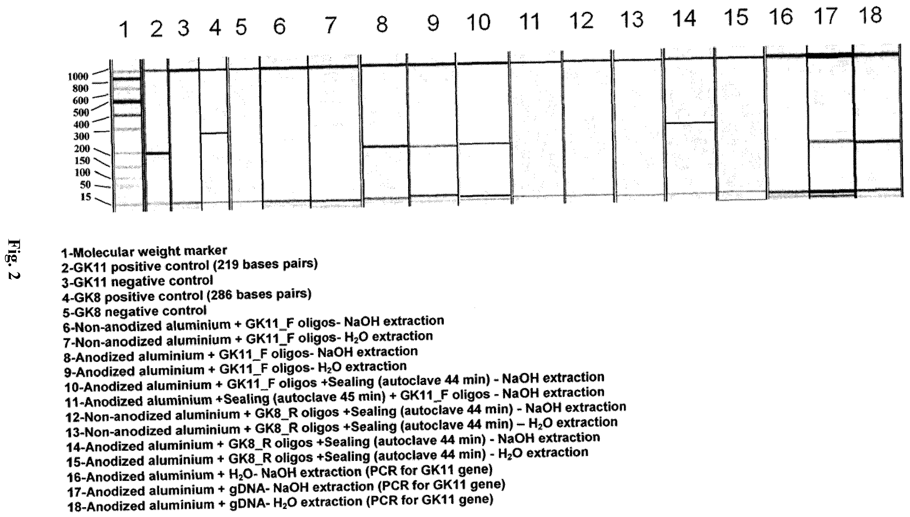

**Figure 2** illustrates the results obtained using different types of DNA and oligos, different eluents for the extraction, for anodized and non-anodised aluminum disks. As can be appreciated, both genomic DNA and oligos were recovered from impregnated anodized aluminum. Lanes 17 and 18 show bands of about 219 bp in size confirming amplification of genomic DNA recovered with both H2O (lane 18) and NaOH (lane 17), but no band for the negative control, i.e. aluminum impregnated only with water (lane 16).

**Figure 2** further confirms recovery of nucleic acids and eliminates almost completely the likelihood of a contamination, i.e. the amplification of a small amount of genomic DNA contaminants. Indeed, the smaller oligos that were used for the impregnation of the aluminum are primers required for the amplifications. Accordingly, to achieve a successful PCR amplification, substantial amounts of primers are required. Briefly, lanes 2-3-4-5 show positive and negative controls for the GK11 and GK8 PCR reactions. As expected, lanes 6, 7, 12 and 13 don't show any bands, since these lanes represent the contact of nucleic acids with a non-anodized aluminum disk. Lanes 8 and 9 confirm that oligonucleotides were incorporated in the disks and later recovered with both, NaOH and H2O. Lanes 10, 14 and 15 demonstrate the impact of sealing to maintain nucleic acids inside the nanopores. No band was detected in lane 15, confirming that H2O was not able to elute oligonucleotides from the sealed disk. However, recovery of both types of oligos was possible when NaOH was used for the recovery as shown with the presence of bands in lanes 10 and 14. Sealing of anodized aluminum prior to incorporation also prevented incorporation of the nucleic (Lane 11).

Taken altogether, these results confirm that small and large nucleic acid molecules can be incorporated or impregnated in the nanopores of the anodized metal and also confirm that such nucleic acids can be retrieved later and that they are still functional. The results also confirm functionality of the sealing which results in preventing impregnation or in preventing elution of the nucleic acids merely with water, NaOH being required.

**EXAMPLE 3: Impregnation/storage, detection and recovery of proteins**

An anodized aluminum plate was impregnated with an aqueous soy solution comprising 50 mg/ml soy proteins and soy lecithins (Swiss natural™ protein soya pro) for 5 min at room temperature. A volume of about 200 $\mu$l of the soy solution was spread on a surface of the disk in the shape of a 1x1 cm plus (+) sign and allowed to dry for 10 min. Next the disk was washed with water for 12 x 30 sec to remove any soy products not incorporated into the porous surface of the disk. After the washing, the disk looked normal with no visible trace of the soy products.

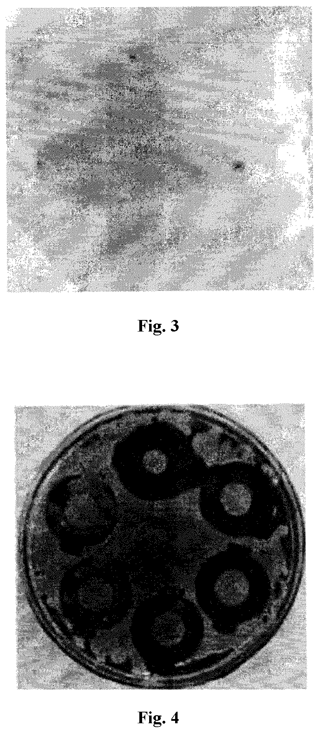

Soy proteins and soy lecithins incorporated within the anodized aluminum disk were revealed using the principles of the Maillard reaction. Briefly, the aluminum disk was heated from underneath with a flame so it reached a temperature of about 110°C-120°C. As expected, the heating caused a Maillard reaction between amine groups of the soy proteins and carbonyls group of the soy lecithin, thereby revealing a brown colored plus sign (+) in the disk (**Figure 3**). The brown coloration could not be observed when using anodized disks sealed prior to contact with the soy solution or when using non-anodized disks (data not shown). When taken altogether, these results confirm that the soy proteins and lecithins were incorporated into the porous layer of the anodized aluminum, and not merely present on the surface of the disk.

**EXAMPLE 4: Impregnation/storage and detection of sugars**

Next, anodized and non-anodized aluminum plates were compared for incorporation of monosaccharides and polysaccharides extracted from red cabbage. Briefly, a red cabbage aqueous extract was obtained by boiling a leaf of cabbage for 10 min in water. The boiled solution was blue in color and, as such, was expected to contain anthocyanin molecules which are water-soluble vacuolar pigments composed of heterosides (i.e. monosaccarides) and aglycone molecules (non-glucidic molecules).

The cooled cabbage solution was cooled to room temperature. Anodized and non-anodized aluminum plates were placed in contact with 2 ml of the cabbage solution for 10 min at room temperature, then washed with ethanol 70% (12 times x 30 sec) and rinsed under water for 5 sec to remove any solution on the surface of the plates. Only the anodized plates were of blue color, confirming that the anthocyanin pigments (monosaccarides) had successfully penetrated the porous layer of the anodized aluminum disks (data not shown).

**EXAMPLE 5: Impregnation/storage, detection and recovery of a chemical dye**

Anodized and non-anodized aluminum disks were compared for incorporation/storage, detection and recovery of a chemical dye. Briefly, anodized and non-anodized disks were prepared as described hereinbefore. The disks were immerged for 10 min in a brilliant blue solution (1,04 % w/v in water). The anodized disks remained colored after the rinsing steps. After their coloration, the disks were sealed (44 min with steam) or not.

The different disks were then submitted to various diffusing conditions to evaluate and compare their potential in diffusing the compounds incorporated into their pores, whether sealed or not. The evaluated conditions were: incubation by soaking in water at room temperature for 24 hours; incubation in peanut oil at room temperature for 24 hours; incubation in a liquid bacterial culture media (MHB media comprising salts, proteins, sugars and various nutritive organic substances) at room temperature for 60 min; and deposition onto agar plates at room temperature for 5 min. For the water and liquid culture media treatment, diffusion was observed visually during incubation (discoloration of disk and coloration of media). For the agar plate treatment, diffusion was observed visually (all chemical dye was transferred from disk to agar plate in 5 min.

Although not shown, all the disks stained with brilliant blue that were sealed remained colored, whatever the tested diffusing conditions. For the non-sealed disks, the diffusion observed was variable, depending on the treatment: Oil: no diffusion was observed for the total duration of the test (24h); Water: no diffusion was visible at 1h but a weak diffusion after 24h; Culture media: the disks were completely discoloured after 15 min (and the media was colored); and Agar plates: the disks were completely discoloured within 5 min (and chemical dye is visible on the agar plate).

These results confirm it is possible to incorporate a chemical dye in the anodized metallic product according to the invention and that sealing is efficacious to avoid diffusion of chemical samples from the nanopores.

**EXAMPLE 6: Impregnation/storage and recovery/detection of an antibacterial**

Anodized and non-anodized aluminum disks were compared for incorporation/storage and recovery/diffusion of a chemical antibacterial compound. Briefly, anodized and non-anodized disks were prepared as described hereinbefore. The disks were then impregnated or not with benzalkonium chloride (an antibacterial) by soaking 10 min at room temperature and rinsed several times with water.

The disks were then tested for diffusion of the antimicrobial benzalkonium by measuring their respective antibacterial activity when deposited for 5 min on an agar plate lawn of S. aureus. As shown in **Figure 4,** the anodized disk impregnated with the benzalkonium chloride solution successfully inhibited bacterial growth, with the subsequent passages on the agar plate reducing the antibacterial activity of the disk (clockwise starting from top). This confirms incorporation and subsequent release of the antibacterial chemical from the anodized porous surface layer.

**EXAMPLE 7: Impregnation/storage and maintenance of biochemical properties of a biological sample**

Anodized plates were compared for testing incorporation/storage and maintenance of biochemical properties of a biological sample. Briefly, a red cabbage aqueous extract (2 ml) as described hereinbefore was placed in contact with anodized and non-anodized aluminum plates for 10 min at room temperature. Next, the plates were rinsed 12 x 30 sec with water. The rinsed anodized plate was of a blue color whereas the non-anodized aluminum plate was not colored (i.e. absence of pores).

It is known that the anthocyanin pigments from the red cabbage may appear in different colors, depending on the pH: the pigments are pink in acidic solutions (pH < 7), purple in neutral solutions (pH ~ 7), greenish-yellow in alkaline solutions (pH > 7), and colourless in very alkaline solutions (the pigments are then completely reduced). Therefore, a change of color may serve as an indicator of functional integrity of the structure and properties of anthocyanin pigments.

After rinsing, a drop of different aqueous solutions (HCl 5% v/v in water for pH 1; biphtalate buffer from Fisher for pH 4, buffer at pH 7 from Labmat, carbonate buffer at pH 10 from LabMat and NaOH 5% w/v in water for pH 14) was deposited on the aluminum plates and the colour was visually assessed. Although not shown, the following colors were observed depending on the pH of the deposited solution: red (pH 1), purple (pH 4), blue (pH 7), green (pH 10) and yellow (pH 14). These color variations are in accordance with the colors predicted in the literature, confirming that the anthocyanin pigments maintained their structural integrity and functional properties after being incorporated into the porous anodized disks.

**EXAMPLE 8: Impregnation/storage and maintenance of functional properties of a chemical compound**

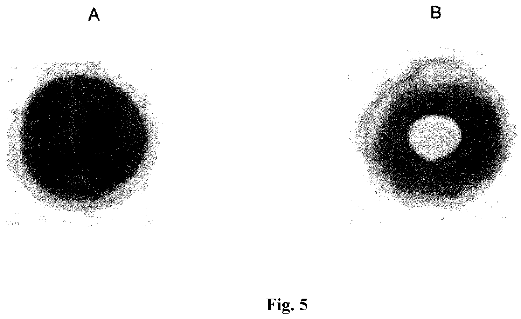

Anodized disks were compared for testing incorporation/storage maintenance of functional properties of chemical compound. Briefly, an anodized disk was prepared as described hereinbefore and immerged for 10 min in a bromophenol blue solution (5% w/v in water) and rinsed (12 x 30 sec. with water). The resulting disk was of a blue color (Figure 5, Picture A).

A drop of a chloridric acid solution (HCl 5% v/v in water) at pH 1 was deposited on the colored aluminum disks and the colour was assessed. As shown in **Figure 5,** a change of color from blue (i.e. dark on Fig. 5A) to yellow (i.e. pale spot on Fig. 5B) was observed at the location where the drop was deposited. This confirms penetration of the acidic solution into the porous layer of the anodized disk and a chemical reaction with the bromophenol blue inside the disk. This further confirms that the bromophenol blue maintained its structural integrity and functional properties when stored in the anodized aluminum disk.

**EXAMPLE 9: Semi-quantitative experiments to evaluate the quantity of samples incorporated in the anodized aluminum disks**

Semi-quantitative experiments were designed to evaluate the quantity of samples incorporated in the anodized aluminum disks of the invention. These experiments were designed to evaluate various factors such as the size (i.e. depth) of the pores, the impregnation technique and time and the presence or absence of sealing.

The experiments were based on: 1) impregnation of anodized aluminum disks with an antimicrobial solution comprising benzalkonium and silver nitrate; and 2) later diffusion of the antimicrobials by depositing the disks on agar plate lawns of S. aureus. As for Example 6, diffusion is measured indirectly by measuring the size of successful inhibition of bacterial growth, with or without successive passages of the disks on the agar plates.

1- The amount of compound stored in the nanopores is directly dependent on the size of the pores

Anodized disks of 22 mm of diameter were prepared as described hereinbefore. Disks were submitted to different anodization periods to obtain a desired thickness of 0, 5, 10, 15 and 20 $\mu$m according the equation provided hereinbefore. The disks were soaked for 30 min at room temperature in an antimicrobial aqueous solution of benzalkonium chloride (2.12 % w/v) and silver nitrate (1.02 % w/v) and rinsed with water.

After the impregnation, disks were tested for diffusion of the antimicrobials. The disks were deposited for 5 min on agar plate lawns of S. aureus, the agar plates were incubated for 24h at 37°C and the zone of inhibition of growth was measured. The results are presented in **Table 2**:

**Table 2: Thickness of the porous layer vs. Inhibition of bacterial growth**

| Thickness of the porous layer (microns) | Size of the zone of inhibition (mm$^2$) | Relative percentage of inhibition* |
|---|---|---|
| 0 | 0 | 0% |
| 5 | 346 | 91% |
| 10 | 346 | 91% |
| 15 | 415 | 109% |
| 20 | 452 | 119% |
| * 100 % inhibition corresponding the surface of the disk | | |

As seen in **Table 2,** the negative control disk (no anodization) did not inhibit bacterial growth. For the 5 $\mu$m and 10 $\mu$m anodized disks, the zone of inhibition is slightly smaller than the size of the disks (91%) indicating a slight diffusion of the antimicrobial from the disk in the agar plate that is nevertheless sufficient to inhibit growth of S. aureus. For the 15 $\mu$m and 20 $\mu$m anodized disks, the zone of inhibition was greater than the size of the disks (109% and 119% respectively), indicating that the amount of antimicrobial compound diffused from the disks was greater. Overall, these results confirm that the thicker is the porous layer, the greater is the amount of compound that may be stored in the nanopores of that porous layer, and the greater is the amount of compound that may be recovered.

2- The amount of stored compound is directly dependent with the duration of the impregnation

Anodized disks of 22 mm in diameter were prepared as described hereinbefore with an anodization period corresponding to a desired thickness of 20 $\mu$m. The disks were soaked at room temperature for different periods (no impregnation, 5 sec, or 5, 10, 20 or 30 min) in an antimicrobial aqueous solution of benzalkonium chloride (2.12 % w/v) and silver nitrate (1.02 % w/v) and rinsed with water.

After the different impregnation periods, the disks were tested for diffusion of the antimicrobials. Briefly, the disks were deposited for 24 successive periods of 5 min each on agar plates lawns of S. aureus, the agar plates were incubated for 24h at 37°C and the zone of inhibition of growth for each deposition was measured and summed up for each impregnation period. The results are presented in **Table 3**:

**Table 3: Duration of the impregnation vs. Inhibition of bacterial growth**

| Duration of the Impregnation | Size of the inhibition zone (cm$^2$) |
|---|---|
| Negative control (no impregnation) | 0 |
| 5 sec | 10,11 |
| 5 min | 17,69 |
| 10 min | 19,93 |
| 20 min | 26,44 |
| 30 min | 33,98 |

As seen in **Table 3,** the size of the zone of inhibition is dependent on the impregnation period, suggesting that a longer impregnation results in a greater quantity of antimicrobial being incorporated in the porous layer of the anodized disks. The duration of the diffusion is also dependent on the quantity of antimicrobial found in the nanopores. It can be calculated that a total of about 25 minutes will be required to completely diffuse all of the antimicrobial incorporated in the pores of the disk during a very short exposition of only 5 sec with the antimicrobial solution, whereas a total of about 90 minutes will be required to achieve a complete diffusion if the disk was placed in contact with the antimicrobial solution for 30 min.

3- Greater sealing results in greater retention of the impregnated compound

Anodized disks of 22 mm of diameter were prepared as described hereinbefore with an anodization period corresponding to a desired thickness of 20 μm. The disks were soaked for different time periods at room temperature in an aqueous antimicrobial solution of benzalkonium chloride (2.12 % w/v) and silver nitrate (1.02 % w/v) followed by soaking in the same antimicrobial solution but at 97°C for the sealing. The time ratio between impregnation and sealing were adjusted to conserve a total time of 74 min. Because it is predicted that a 20 μm-thick disk will be sealed at 100% when incubated for 44 min, shorter time periods will result in lower degrees of sealing (ranked herein from 0 (no sealing) to 4 (100% sealing efficacy)). So, to obtain 100% sealing efficacy, the disk was soaked for 30 min in an antimicrobial solution at room temperature and 44 min in an antimicrobial solution at 97°C. For 0% of sealing, disk was soaked for 74 min in antimicrobial solution at room temperature.

After the various impregnation/sealing and rinsing steps, the disks were tested for diffusion of the antimicrobials. Briefly, the disks were deposited for 6 successive periods of 5 min each on agar plates lawns of S. aureus, the agar plates were incubated for 24h at 37°C and the zone of inhibition of growth for each deposition was measured and summed up for each impregnation/sealing period. The results are presented in **Table 4:**

**Table 4: Level of sealing vs. Inhibition of bacterial growth**

| Antimicrobial impregnation at room temperature (time in minute) | Antimicrobial impregnation at 97°C for sealing (time in minute) | Relative level of sealing | Size of the zone of inhibition (cm$^2$) | Relative percentage of inhibition* |
|---|---|---|---|---|
| Negative control (no impregnation) | Negative control (no impregnation) | - | 0 | - |
| 74 | 0 | 0 | 9,82 | 100% |
| 63 | 11 | 1 | 5,44 | 55% |
| 52 | 22 | 2 | 3,18 | 32% |
| 41 | 33 | 3 | 3,18 | 32% |
| 30 | 44 | 4 | 1,52 | 15% |
| *100% inhibition corresponding to the non-sealed disks | | | | |

As seen in **Table 4,** the size of the zone of inhibition is indirectly proportional to the relative degree of sealing. When the disks were sealed completely (level 4), the measured zone of inhibition was minimal (only 15%), suggesting that diffusion of the antimicrobial from the pores was more difficult. However, diffusion was easier when the disks were not sealed (level 0) or sealed to a lesser degree (from 55% to 32% growth inhibition for levels 1-3 respectively).

Therefore, these results suggest that, under the same conditions and for the same period of time, the diffusion will be less effective (longer time, less complete) if the nanopores of the porous layer have been sealed.

**EXAMPLE 10: Long term conservation of samples**

An experiment was designed to evaluate the presence and integrity of samples stored in the anodized metallic support.

Firstly, it was still possible to detect the presence of nucleic acids in anodized aluminum after three years of storage inside anodized aluminum disks. The stored nucleic acids (genomic DNA and oligos) were visualized following coloration with ethidium bromide and detection under UV light (results not shown).

Secondly, it was still possible to measure antibacterial activity of anodized aluminum disks impregnated 4 years ago with benzalkonium chloride (2.12 % w/v). The antimicrobial activity was measured by diffusion of the antimicrobial benzalkonium following deposition for 5 min on an agar plate lawn of S. aureus (results not shown).

Headings are included herein for reference and to aid in locating certain sections. These headings are not intended to limit the scope of the concepts described therein, and these concepts may have applicability in other sections throughout the entire specification.

As used herein and in the appended claims, the singular forms "a", "an", and "the" include plural referents unless the context clearly indicates otherwise.

Unless otherwise indicated, all numbers expressing quantities of ingredients, reaction conditions, concentrations, properties, and so forth used in the specification and claims are to be understood as being modified in all instances by the term "about". At the very least, each numerical parameter should at least be construed in light of the number of reported significant digits and by applying ordinary rounding techniques. Accordingly, unless indicated to the contrary, the numerical parameters set forth in the present specification and attached claims are approximations that may vary depending upon the properties sought to be obtained. Notwithstanding that the numerical ranges and parameters setting forth the broad scope of the embodiments are approximations, the numerical values set forth in the specific examples are reported as precisely as possible. Any numerical value, however, inherently contain certain errors resulting from variations in experiments, testing measurements, statistical analyses and such.

It is understood that the examples and embodiments described herein are for illustrative purposes only and that various modifications or changes in light thereof will be suggested to persons skilled in the art. The scope of the claims should not be limited by these embodiments but should be given the broadest interpretation consistent with the description as a whole.

## Claims

1. A traceable metallic product (1), wherein said metallic product is selected from the group consisting of aluminum, titanium, zinc, magnesium, niobium, tantalum and anodizable alloys thereof, wherein said metallic product comprises a metallic porous surface layer (3) formed by anodization, and wherein said porous surface layer (3) comprises nanopores (4) and at least one traceable biological compound (5) carried in said nanopores (4), wherein said at least one traceable biological compound is recoverable in an integral form from said porous surface layer for detection, identification and/or utilization purposes, and wherein said at least one traceable biological compound is selected from the group consisting of nucleic acids, peptidic molecules, lipids, mono and polysaccharides, hormones, and vitamins.

2. The traceable metallic product (1) according to claim 1, wherein said porous surface (3) layer further comprises a sealant.

3. The traceable metallic product (1) according to claim 1 or 2, wherein said porous surface layer (3) further comprises an electrodeposit of at least one metal selected from the group consisting of silver, gold, copper, nickel, zinc, tin, cadmium, palladium and platinum.

4. The traceable metallic product (1) according to anyone of claims 1 to 3, wherein said traceable metallic product consists of a traceable piece of aluminum comprising a porous surface layer (3) formed by anodization, and wherein said porous surface layer comprises nucleic acid molecules inside pores of the surface layer.

5. An article of manufacture incorporating a traceable metallic product (1) according to anyone of claims 1 to 4, wherein said article of manufacture is selected from the group consisting of plane parts, automotive vehicle parts, train parts, boat parts, electronic and computer components, military-related products, medical devices, jewelry, art works, products labels, keys, food containers, credit cards, money, collectibles, and casino equipment.

6. The article of manufacture according to claim 5, wherein said article consists of a metallic support for storing traceable biological compounds in the nanopores and recovery for later detection, identification and/or utilization.

7. Use of at least one traceable biological compound (5) for tracking an anodized metallic product (1) comprising a porous layer (3), wherein said at least one traceable biological compound (5) is found inside pores (4) of the surface layer (3), and wherein said at least one traceable biological compound is recoverable in an integral form from said porous surface layer for detection, identification and/or utilization purposes.

8. A method for obtaining the traceable metallic product (1) of any one of claims 1 to 4, comprising the steps of:

- providing an anodized metallic product having a porous surface layer formed by anodization; and
- anodizing said metallic substrate (1) for obtaining a metallic product having a porous surface layer (3) formed by anodization; and
- impregnating a region of said porous surface layer (3) with said at least one traceable biological compound (5) wherein said at least one traceable biological compound is recoverable in an integral form from said porous surface layer for detection, identification and/or utilization purposes.

9. The method according to claim **8,** further comprising recovering said traceable biological compound (5) from said porous surface layer (3) for detection, identification and/or utilization purposes.

10. The method according to claim **8** or claim **9,** further comprising a sealing or clogging step which is carried out before, simultaneously or after impregnation.

11. The method according to anyone of claims **8** to **10,** further comprising a dying step before, simultaneously or after impregnating the porous layer with said at least one traceable biological compound.

12. A method for tracking a porous metallic product (1) having a porous surface layer (3) formed by anodization, comprising:

    - assigning at least one predetermined compound (5) to a porous metallic product (1) to be tracked;
    - impregnating a region of said porous surface layer (3) with said at least one predetermined compound (5);
    - tracking said porous metallic product later in time by detecting and/or identifying said at least one predetermined compound (5) and by correlating the presence and/or identity of said at least one predetermined compound with said assigning,
    wherein said at least one predetermined compound is recoverable in an integral form from said porous surface layer for said detecting and/or identifying; and wherein a positive detection or identification provides a positive verification and traceability of the metallic product to be tracked.

13. The method according to claim **12,** further comprising recovering said at least one predetermined compound (5) from said porous surface layer (3) for detection, identification and/or utilization purposes.

14. The method according to claim **12** or claim **13,** wherein the predetermined compound (5) is a traceable biological compound selected from the group consisting of nucleic acids, peptidic molecules, lipids, mono and polysaccharides, hormones, and vitamins.

15. The method according to anyone of claims **12** to **14,** wherein the predetermined compound (5) is a chemical compound selected from the group consisting of reactants, colorants, fluorescent products, phosphorescent products, antibacterials, antivirals, antibiotics, antifungals, odorants, and gustative compounds.

16. A method for nanostorage of a traceable biological compound on a metallic support (1), comprising the steps of:

    - providing an anodized metallic support (1) having a porous surface layer (3) formed by anodization and which is integral to the metallic product (1), wherein said metallic product (1) is selected from the group consisting of aluminum, titanium, zinc, magnesium, niobium, tantalum and anodizable alloys thereof; and
    - impregnating a region of the porous layer (3) with at least one traceable biological compound (5) to be stored; and
    - storing said at least one traceable biological compound (5) in said anodized metallic support (1).
    wherein said at least one traceable biological compound (5) is recoverable in an integral form from said porous surface layer (3) for later analysis or use.

**Fig. 1**

**Fig. 2**

1-Molecular weight marker
2-GK11 positive control (219 bases pairs)
3-GK11 negative control
4-GK8 positive control (286 bases pairs)
5-GK8 negative control
6-Non-anodized aluminium + GK11_F oligos- NaOH extraction
7-Non-anodized aluminium + GK11_F oligos- $H_2O$ extraction
8-Anodized aluminium + GK11_F oligos- NaOH extraction
9-Anodized aluminium + GK11_F oligos- $H_2O$ extraction
10-Anodized aluminium + GK11_F oligos +Sealing (autoclave 44 min) - NaOH extraction
11-Anodized aluminium +Sealing (autoclave 45 min) + GK11_F oligos - NaOH extraction
12-Non-anodized aluminium + GK8_R oligos +Sealing (autoclave 44 min) – NaOH extraction
13-Non-anodized aluminium + GK8_R oligos +Sealing (autoclave 44 min) – $H_2O$ extraction
14-Anodized aluminium + GK8_R oligos +Sealing (autoclave 44 min) - NaOH extraction
15-Anodized aluminium + GK8_R oligos +Sealing (autoclave 44 min) - $H_2O$ extraction
16-Anodized aluminium + $H_2O$- NaOH extraction (PCR for GK11 gene)
17-Anodized aluminium + gDNA- NaOH extraction (PCR for GK11 gene)
18-Anodized aluminium + gDNA- $H_2O$ extraction (PCR for GK11 gene)

19

**Fig. 3**

**Fig. 4**

A    B

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5124172 A
- US 7736724 B
- US 7361471 B

### Non-patent literature cited in the description

- Chromate-Free Method of Preparing Aluminum Surfaces for Adhesive Bonding. **RUSSELL ; GARNIS.** An Etchant Composition of Low Toxicity. Army Armament Research and Development Center, Dover NJ, Large Caliber Weapon Systems Lab, 1977
- Equation taken from: Anodizing and coloring of aluminum alloys. ASM International, 2002, 170
- **SATOSHI KAWAY.** Anodizing and coloring of aluminum alloys. Finishing Publications, 2002, 159